(19) Europäisches Patentamt
European Patent Office
Office européen des brevets

(11) EP 0 806 415 B1

(12) # EUROPÄISCHE PATENTSCHRIFT

(45) Veröffentlichungstag und Bekanntmachung des Hinweises auf die Patenterteilung:
**16.02.2000 Patentblatt 2000/07**

(51) Int Cl.[7]: **C07D 213/81**, C07D 401/12, A01N 43/40, A01N 43/56

(21) Anmeldenummer: **97106080.1**

(22) Anmeldetag: **14.04.1997**

(54) **Verfahren zur Herstellung von Arylamiden heteroaromatischer Carbonsäuren**

Process for the preparation of arylamides of heteroaromatic carboxylic acids

Procédé pour la préparation d'arylamides d'acides carboxyliques hétéroaromatiques

(84) Benannte Vertragsstaaten:
**AT BE CH DE DK ES FI FR GB GR IE IT LI LU MC NL PT SE**
Benannte Erstreckungsstaaten:
**AL LT LV RO SI**

(30) Priorität: **09.05.1996 CH 117896**

(43) Veröffentlichungstag der Anmeldung:
**12.11.1997 Patentblatt 1997/46**

(73) Patentinhaber: **LONZA AG**
**CH-3945 Gampel/Wallis (CH)**

(72) Erfinder:
- **Roduit, Jean-Paul, Dr.**
  **3979 Grône, Kanton Wallis (CH)**
- **Kalbermatten, Georges**
  **3938 Ausserberg, Kanton Wallis (CH)**

(74) Vertreter: **Winter, Brandl, Fürniss, Hübner, Röss, Kaiser, Polte, Kindermann Partnerschaft**
**Patent- und Rechtsanwaltskanzlei**
**Alois-Steinecker-Strasse 22**
**85354 Freising (DE)**

(56) Entgegenhaltungen:
**EP-A- 0 053 011**
**EP-A- 0 447 004**
**EP-A- 0 582 825**
**WO-A-94/27974**

- **JOURNAL OF THE AMERICAN CHEMICAL SOCIETY, Bd. 111, Nr. 23, 1989, Seiten 8742-8744, XP000670254 BEN-DAVID Y ET AL: "CHELATE-ASSISTED, PD-CATALYZED EFFICIENT CARBONYLATION OF ARYL CHLORIDES"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Verfahren zur Herstellung von Arylamiden heteroaromatischer Carbonsäuren durch Umsetzung von heteroaromatischen Halogenverbindungen mit Kohlenmonoxid und aromatischen Aminen in Gegenwart eines Katalysators und einer Base. Sie betrifft weiterhin ein neues Halogenpyridin als Ausgangsmaterial für die erfindungsgemässe Herstellung eines Arylamids.

Die erfindungsgemäss herstellbaren Amide besitzen die allgemeine Formel

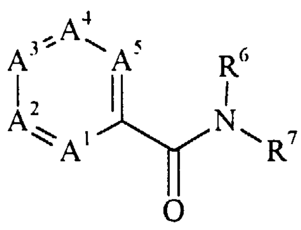

I.

Hierin bedeuten:

$A^1$ Stickstoff oder $CR^1$,
$A^2$ Stickstoff oder $CR^2$,
$A^3$ Stickstoff oder $CR^3$,
$A^4$ Stickstoff oder $CR^4$,

und $A^5$ Stickstoff oder $CR^5$,
mit der Massgabe dass wenigstens eines der Ringglieder $A^1$ bis $A^5$ Stickstoff ist und nicht zwei Stickstoffatome unmittelbar miteinander verbunden sind;

$R^1$ bis $R^5$, soweit vorhanden, unabhängig voneinander Wasserstoff, $C_{1-4}$-Alkyl oder Aryl, wobei jedoch einer der Substituenten $R^1$ bis $R^5$ eine Gruppe der Formel -OR ist, in welcher R ein gegebenenfalls substituierter aromatischer oder heteroaromatischer Rest ist;
$R^6$ Wasserstoff oder $C_{1-4}$-Alkyl
und $R^7$ einen gegebenenfalls substituierten aromatischen oder heteroaromatischen Rest. Insbesondere gehören hierzu die Arylamide der Pyridin-, Pyrimidin-, Pyrazin- und 1,3,5-Triazincarbonsäuren.

Zahlreiche Verbindungen dieser Struktur, insbesondere solche, bei denen einer der Substituenten $R^1$ bis $R^5$ eine Aryloxygruppe (-OR) in Nachbarschaft zu einem Ringstickstoffatom ist, sind wichtige Herbizide (WO-A 94/27974, EP-A 0 053 011, EP-A 0 447 004). Ihre Synthese geht üblicherweise von den entsprechenden Carbonsäuren oder Carbonsäurederivaten (Säurechloride, Ester, Nitrile) aus. Diese sind jedoch oft schwer zugänglich und deshalb teuer.

Die EP-A-0 582 825 beschreibt die Herstellung von 5-Chlorpyridin-2-carboxamiden durch Umsetzung von 2,5-Dichlorpyridin mit Kohlenmonoxid und Aminoverbindungen wie Ethylendiamin in Gegenwart von Palladium-Phosphin-Katalysatoren.

Aufgabe der vorliegenden Erfindung war es, ein alternatives Verfahren zur Herstellung von Verbindungen der allgemeinen Formel I zur Verfügung zu stellen, welches von leichter zugänglichen Edukten ausgeht.

Erfindungsgemäss wird diese Aufgabe durch das Verfahren nach Patentanspruch 1 gelöst. Es wurde gefunden, dass Halogenverbindungen der allgemeinen Formel

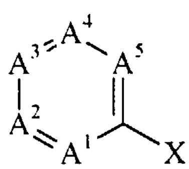

II,

worin $A^1$ bis $A^5$ die oben genannten Bedeutungen haben und X Chlor, Brom oder Iod ist, mit Kohlenmonoxid und einem primären oder sekundären Amin der allgemeinen Formel

$$R^6\text{-NH-}R^7 \qquad III,$$

worin $R^6$ und $R^7$ die oben genannten Bedeutungen haben, in Gegenwart einer Base in guter bis fast quantitativer Ausbeute direkt zu den gewünschten Produkten (I) reagieren, wenn als Katalysator ein Komplex von Palladium mit einem Triphenylphosphin der allgemeinen Formel

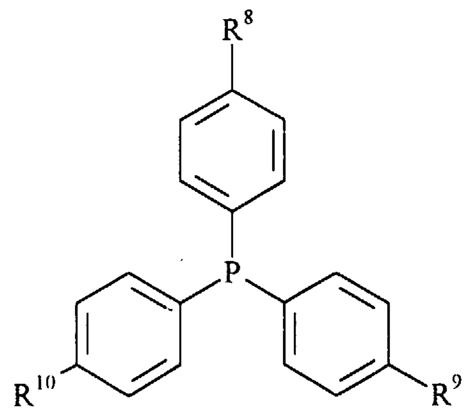

IV,

worin $R^8$ bis $R^{10}$ unabhängig voneinander $C_{1-4}$-Alkyl, $C_{1-4}$-Alkoxy oder Fluor bedeuten, anwesend ist. Als wesentlich hat sich hierbei die Anwesenheit der Substituenten $R^8$ bis $R^{10}$ in Position 4 (para-Position) der Phenylgruppen erwiesen. Die entsprechenden ortho- und meta-substituierten Verbindungen ergeben ebenso wie das unsubstituierte Triphenylphosphin erheblich schlechtere Ausbeuten oder überhaupt kein Produkt.

Unter $C_{1-4}$-Alkyl sind hier und im folgenden alle linearen oder verzweigten primären, sekundären oder tertiären Alkylgruppen mit bis zu 4 Kohlenstoffatomen zu verstehen.

Unter aromatischen oder heteroaromatischen Resten sind hier und im folgenden insbesondere mono- oder polycyclische Systeme wie beispielsweise Phenyl, Naphthyl, Biphenylyl, Anthracenyl, Furyl, Pyrrolyl, Pyrazolyl, Thiophenyl, Pyridyl, Indolyl oder Chinolinyl zu verstehen. Diese können einen oder mehrere gleiche oder verschiedene Substituenten tragen, beispielsweise niedrige Alkylgruppen wie Methyl, halogenierte Alkylgruppen wie Trifluormethyl, niedrige Alkoxygruppen wie Methoxy, oder niedrige Alkylthio- (Alkansulfanyl-) oder Alkansulfonylgruppen wie Methylthio oder Ethansulfonyl. Unter substituiertem Phenyl sind insbesondere Gruppen wie (*p*-) Fluorphenyl, (*p*-) Methoxyphenyl, (*p*-) Tolyl oder (*p*-) Trifluormethylphenyl zu verstehen.

Die als Ausgangsmaterial dienenden Halogenverbindungen (II) sind entweder bekannte Verbindungen oder sie können analog zu bekannten Verbindungen hergestellt werden. Zahlreiche derartige Verbindungen sind beispielsweise in US-A 4 254 125 und in EP-A 0 001 187 offenbart. Die Verbindung 2-Chlor-6-[1-methyl-3-(trifluormethyl)pyrazol-5-yloxy]pyridin ist neu und ebenfalls Gegenstand der vorliegenden Erfindung.

Vorzugsweise eignet sich das erfindungsgemässe Verfahren zur Herstellung solcher Amide (I), in denen $A^2$ Stickstoff ist und mit den übrigen Ringgliedern einen Pyridinring bildet.

Besonders bevorzugt sind dabei jene Amide (I), in denen $R^1$ eine Gruppe der Formel -OR ist, wobei R die oben genannte Bedeutung hat.
Ebenfalls bevorzugt sind solche Amide (I), in denen $A^1$ Stickstoff ist und mit den übrigen Ringgliedern einen Pyridinring bildet,
solche, in denen $A^1$ und $A^5$ Stickstoff sind und mit den übrigen Ringgliedern einen Pyrimibilden,
solche, in denen $A^1$ und $A^4$ Stickstoff sind und mit den übrigen Ringgliedern einen Pyrazinring bilden,
sowie solche, in denen $A^1$, $A^3$ und $A^5$ Stickstoff sind und mit den übrigen Ringgliedern einen 1,3,5-Triazinring bilden.

Unter den vier letztgenannten Klassen sind wiederum diejenigen Amide besonders bevorzugt, in welchen $R^2$ eine Gruppe der Formel -OR ist, wobei R die oben genannte Bedeutung hat.

Unter den Amiden (I) sind ausserdem diejenigen bevorzugt, in denen R eine gegebenenfalls substituierte Phenylgruppe ist. Dies gilt insbesondere für die vorstehend genannten Amide mit Pyridin-, Pyrimidin-, Pyrazin- oder 1,3,5-Triazinring, in denen $R^1$ oder $R^2$ eine Gruppe der Formel -OR ist.

Ebenfalls bevorzugt sind solche Amide, in denen $R^6$ Wasserstoff und $R^7$ eine gegebenenfalls substituierte

Phenylgruppe ist.

Als Halogenverbindungen (II) sind die Chlorverbindungen (X = Cl) bevorzugt.

Besonders bevorzugte Triphenylphosphine (IV) sind diejenigen, in denen $R^8$ bis $R^{10}$ gleich und $C_{1-4}$-Alkoxygruppen sind.

Ganz besonders bevorzugt ist das Triphenylphosphin, in dem $R^8$ bis $R^{10}$ Methoxygruppen sind.

Der katalytisch wirksame Palladium-Phosphin-Komplex wird vorteilhaft *in situ* gebildet, indem Palladium in fein verteilter elementarer Form (z. B. Palladium auf Aktivkohle), ein Pd(II) Salz (z. B. das Chlorid oder das Acetat), oder ein geeigneter Pd(II)-Komplex (z. B. Dichloro-bis(triphenylphosphin)palladium(II)) mit dem Phosphin zur Reaktion gebracht wird. Besonders bevorzugt als Palladiumquelle ist das Palladium(II)acetat. Das Palladium wird vorzugsweise in einer Menge von 0,02 bis 0,2 Mol-% Pd(II) oder 0,5 bis 2 Mol-% Pd(0) (als Pd/C), jeweils bezogen auf die Halogenverbindung (II), eingesetzt. Das Phosphin wird vorteilhaft im Überschuss (bezogen auf Pd) eingesetzt, vorzugsweise in einer Menge von 0,2 bis 5 Mol-%, ebenfalls bezogen auf die Halogenverbindung (II). Als Lösungsmittel können sowohl relativ unpolare, wie beispielsweise Toluol oder Xylol, als auch polare wie beispielsweise Acetonitril, Tetrahydrofuran oder *N,N*-Dimethylacetamid eingesetzt werden.

Als Base wird vorzugsweise eine relativ schwache Base eingesetzt. Diese braucht in dem verwendeten Lösungsmittel nicht löslich zu sein. Gut geeignet sind beispielsweise Carbonate wie Natriumcarbonat oder Kaliumcarbonat oder Acetate wie Natriumacetat. Mit der letztgenannten Base wurden ganz besonders gute Ergebnisse erzielt.

Die Reaktionstemperatur liegt vorzugsweise bei 80 bis 250 °C.

Der Kohlenmonoxiddruck liegt vorzugsweise bei 1 bis 50 bar.

Die Halogenverbindungen (II) werden vorteilhaft dadurch hergestellt, dass ein Dihalogenid der allgemeinen Formel

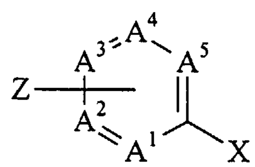

V,

worin X Chlor, Brom oder Iod ist und $A^1$ bis $A^5$ die oben genannten Bedeutungen haben, mit der Massgabe, dass einer der Reste $R^1$ bis $R^5$ an einem zu einem Ringstickstoffatom benachbarten Kohlenstoffatom Z ist, wobei Z Chlor, Brom oder Iod bedeutet und die übrigen von $R^1$ bis $R^5$, soweit vorhanden, die in Anspruch 1 genannte Bedeutung haben, mit einer aromatischen oder heteroaromatischen Hydroxyverbindung der allgemeinen Formel

$$R\text{-}OH \qquad VI,$$

worin R die in Anspruch 1 genannte Bedeutung hat, umgesetzt wird.

Die folgenden Beispiele verdeutlichen die Durchführung des erfindungsgemässen Verfahrens.

**Beispiel 1**

**2-Chlor-6-[3-(trifluormethyl)phenoxy]pyridin**

In 420 ml *N,N*-Dimethylacetamid wurden 17,45 g (690 mmol) Natriumhydrid (95%) suspendiert. Bei 15 °C wurden innerhalb von 2 h 106,7 g (658 mmol) 3-(Trifluormethyl)phenol zugetropft. Die so erhaltene Phenolatlösung wurde innerhalb von 2,5 h unter Stickstoff zu einer auf 90 °C erwärmten Lösung von 162,4 g (1,097 mol) 2,6-Dichlorpyridin in 330 ml *N,N*-Dimethylacetamid getropft. Nach weiteren 3 h Reaktionszeit wurde das Gemisch auf Raumtemperatur abgekühlt, das ausgefallene Natriumchlorid abfiltriert und das Filtrat eingeengt. Der Rückstand wurde mit Toluol und 0,1 N Salzsäure aufgenommen, die organische Phase mit gesättigter Natriumchloridlösung gewaschen und eingeengt. Der ölige Rückstand (ca. 200 g) wurde im Vakuum destilliert.

Ausbeute: 151,5 g (84%) farbloses Öl, Gehalt (GC) 99,8%

$n_D^{20}$ = 1,5267

MS; *m/z*: 273/275; 238; 39

| | |
|---|---|
| $^1H$ NMR ($CDCl_3$): δ = | 6,84 (d, *J*= 7,8 Hz, 1H); 7,07 (d, *J*= 7,8 Hz, 1H); 7,35 (m, 1H); 7,42 (m, 1H); 7,45-7,52 (m, 2H); 7,65 (t, *J*= 7,8 Hz, 1H). |
| $^{13}C$ NMR ($CDCl_3$): δ = | 109,88 (CH); 118,16 (CH); 119,24 (CH); 121,67 (CH); 123,74 ($CF_3$); 124,50 (CH); 130,24 (CH); 132,21 (*C*$CF_3$); 141,77 (CH); 149,12 (C); 153,89 (C); 162,28 (C). |

**Beispiel 2**

**3-Chlor-2-[3-(trifluormethyl)phenoxy]pyridin**

Unter Stickstoff wurden 7,68 g Natriumhydrid-Dispersion (ca. 50 % in Mineralöl) mit Pentan gewaschen, dann wurden 100 ml *N,N*-Dimethylformamid zugegeben. Bei Raumtemperatur wurden innerhalb von 30 min 21,92 g (135 mmol) 3-(Trifluormethyl)phenol zugetropft. Die so erhaltene Phenolatlösung wurde innerhalb von 2 h unter Stickstoff zu einer auf 120 °C erhitzten Lösung von 20,1 g (136 mmol) 2,3-Dichlorpyridin in 80 ml *N,N*-Dimethylformamid getropft. Nach 3 h Reaktionsdauer wurde das Gemisch auf Raumtemperatur abgekühlt, das ausgefallene Natriumchlorid abfiltriert und das Filtrat eingeengt. Der Rückstand wurde mit Toluol und 0,1 N Salzsäure extrahiert, die organische Phase mit gesättigter Natriumchloridlösung gewaschen und eingeengt. Der ölige Rückstand wurde im Vakuum destilliert.

Ausbeute: 24,75 g (67%) farbloses Öl, Gehalt (GC) 99,7%

Sdp.$_{18\ mbar}$ = 145-148 °C

$n_D^{20}$ = 1,5282

MS; *m/z*: 273/275

| | |
|---|---|
| $^1H$ NMR ($CDCl_3$): δ = | 6,99 (m, 1H); 7,36 (d, 1H); 7,45-7,53 (m, 3H); 7,77 (d, 1H); 8,02 (d, 1H). |
| $^{13}C$ NMR ($CDCl_3$): δ = | 118,66 (CH); 119,44 (C); 119,98 (CH); 121,75 (CH); 123,78 ($CF_3$); 124,94 (CH); 130,13 (CH); 132,16 (*C*$CF_3$); 139,65 (CH); 145,20 (CH); 153,88 (C); 158,51 (C). |

**Beispiel 3**

***N*-(4-Fluorphenyl)-6-[3-(trifluormethyl)phenoxy]pyridin-2-carboxamid**

In einem Autoklaven wurden bei Raumtemperatur 10,26 g (37,5 mmol) 2-Chlor-6-[3-(trifluormethyl)phenoxy]pyridin (Gehalt 99,5%, hergestellt nach Beispiel 1), 6,25 g (56,2 mmol) 4-Fluoranilin, 4,37 g (41,3 mmol) Natriumcarbonat, 26,3 mg (37,5 μmol) Dichloro-bis(triphenylphosphin)palladium(II) und 0,40 g (1,125 mmol) Tris(4-methoxyphenyl)phosphin (IV, $R^8$ = $R^9$ = $R^{10}$ = Methoxy) in 37,5 ml Xylol vorgelegt. Der Autoklav wurde mit Inertgas gespült, dann wurden 5 bar Kohlenmonoxid aufgepresst und auf 150 °C aufgeheizt. Der CO-Druck wurde auf 18 bar erhöht und das Gemisch 21 h bei 150 °C gerührt. Nach dem Abkühlen auf Raumtemperatur und der Druckentlastung wurde das Reaktionsgemisch mit je 50 ml Xylol und Wasser versetzt und filtriert. Die wässrige Phase wurde mit 25 ml Xylol extrahiert und die vereinigten organischen Phasen mit 30 ml Wasser gewaschen. In der Xylol-Phase waren mittels GC weder unumgesetztes Edukt noch Nebenprodukte nachzuweisen. Nach dem Abdestillieren des Lösungsmittels wurde das Rohprodukt (15,83 g) als gelber Feststoff erhalten.
Zur Reinigung wurde das Rohprodukt aus Methylcyclohexan umkristallisiert.

Ausbeute: 12,13 g (86%) leicht beiger Feststoff

Schmp.: 103-104,5 °C

MS; *m/z*: 376 ($M^+$), 238

$^1H$ NMR ($CDCl_3$): δ = 6,99-7,04 (m, 2H); 7,17 (d, *J*= 8,4 Hz, 1H); 7,40 (m, 1H); 7,46-7,51 (m, 2H); 7,55-7,63 (m, 3H); 7,93 (t, *J*= 7,8 Hz, 1H); 8,03 (d, *J*= 7,8 Hz, 1H); 9,24 (br. m, 1H).

**Beispiel 4**

***N*-(4-Fluorphenyl)-6-[3-(trifluormethyl)phenoxy]pyridin-2-carboxamid**

Es wurde verfahren wie in Beispiel 3 beschrieben, jedoch wurde anstelle von Dichloro-bis-(triphenylphosphin) palladium(II) die gleiche molare Menge Palladium(II)acetat eingesetzt. Der CO-Druck betrug 19 bar. Erhalten wurden 15,77 g Rohprodukt und nach Umkristallisation 11,82 g (83,8%) farbloser Feststoff.
Schmp.: 104-105 °C

**Beispiel 5**

***N*-(4-Fluorphenyl)-6-[3-(trifluormethyl)phenoxy]pyridin-2-carboxamid**

Es wurde verfahren wie in Beispiel 3 beschrieben, jedoch wurde anstelle des Tris(4-methoxyphenyl)phosphins die gleiche molare Menge Tris(4-methylphenyl)phosphin (IV, $R^8$ = $R^9$ = $R^{10}$ = Methyl) eingesetzt. Der CO-Druck betrug 20 bar, die Reaktionsdauer 21 h. Mittels GC wurde die Zusammensetzung der gelösten Produkte in der Xylol-Phase bestimmt. Gefunden wurden 94,1% Titelverbindung und 5,9% Edukt.

**Beispiel 6**

***N*-(4-Fluorphenyl)-6-[3-(trifluormethyl)phenoxy]pyridin-2-carboxamid**

Es wurde verfahren wie in Beispiel 3 beschrieben, jedoch wurde anstelle des Tris(4-methoxyphenyl)phosphins die gleiche molare Menge Tris(4-fluorphenyl)phosphin (IV, $R^8$ = $R^9$ = $R^{10}$ = Fluor) eingesetzt. Der CO-Druck betrug 19 bar, die Reaktionsdauer 21 h. Mittels GC wurde die Zusammensetzung der gelösten Produkte in der Xylol-Phase bestimmt. Gefunden wurden 90,8% Titelverbindung und 9,2% Edukt.

**Beispiel 7**

**2-Chlor-6-[1-methyl-3-(trifluormethyl)pyrazol-5-yloxy]pyridin**

In 25 ml *N,N*-Dimethylacetamid wurden 1,8 g (71 mmol) Natriumhydrid (95%) suspendiert. Bei 22 °C wurde innerhalb von 1 h eine Lösung von 12,46 g (67 mmol) 1-Methyl-5-hydroxy-3-(trifluormethyl)pyrazol (*J. Heterocycl. Chem.* **1990**, *27*, 243) in 40 ml *N,N*-Dimethylacetamid zugetropft. Die so erhaltene Lösung wurde innerhalb von 3 h unter Stickstoff zu einer auf 135 °C erwärmten Lösung von 18,5 g (125 mmol) 2,6-Dichlorpyridin in 37,5 ml *N,N*-Dimethylacetamid getropft. Nach weiteren 5 h Reaktionszeit wurde das Gemisch auf Raumtemperatur abgekühlt, das ausgefallene Natriumchlorid abfiltriert und das Filtrat eingeengt. Der Rückstand wurde mit Toluol und Wasser aufgenommen, die organische Phase mit gesättigter Natriumchloridlösung gewaschen und eingeengt. Der feste Rückstand wurde im Vakuum destilliert. Die Titelverbindung destilliert bei 175 °C unter 25 mbar. So wurden 12,5 g Rohprodukt als gelber Feststoff erhalten (96 % Reinheit, GC). Zur Reinigung wurde aus Diisopropylether umkristallisiert.

Ausbeute: 11,55 g (62%) farblose Kristalle, Gehalt (GC) 99,9%
Schmp.: 56-58°C
MS; *m*/*z*: 277/279

$^1H$ NMR ($CDCl_3$): δ = 3,80 (s, 3H); 6,40 (s, 1H); 6,96 (d, *J* = 8,2 Hz, 1H); 7,18 (d, *J* = 8,2 Hz, 1H); 7,74 (t, *J* = 8,2 Hz, 1H).

**Beispiel 8**

***N*-(4-Fluorphenyl)-6-[1-methyl-3-(trifluormethyl)pyrazol-5-yloxy]pyridin-2-carboxamid**

Es wurde verfahren wie in Beispiel 4 beschrieben. Aus 3,47 g (12,5 mmol) 2-Chlor-6-[1-methyl-3-(trifluormethyl)pyrazol-5-yloxy]pyridin, 2,08 g (18,7 mmol) 4-Fluoranilin, 1,46 g (13,8 mmol) Natriumcarbonat, 5,6 mg (25 µmol) Palladium(II)acetat und 0,13 g (375 µmol) Tris(4-methoxyphenyl)phosphin in 12,5 ml Xylol wurden nach 21 h bei 150 °C unter 19 bar CO Druck (GC: vollständiger Umsatz) 4,92 g Rohprodukt als hellgelber Feststoff erhalten. Zur Reinigung wurde aus Methylcyclohexan umkristallisiert.

Ausbeute: 3,97 g (84,4%) leicht beige Kristalle
Schmp. 138-139 °C

$^1$H NMR ($CDCl_3$): δ = 3,85 (s, 3H); 6,41 (s, 1H); 7,06 (m, 2H); 7,29 (d, *J* = 8,1 Hz, 1H); 7,59 (m, 2H); 8,05 (t, *J* = 8,1 Hz, 1H); 8,14 (d, *J* = 8,1 Hz, 1H); 9,28 (bs, 1H).

**Vergleichsbeispiel 1**

Es wurde analog zu Beispiel 3 verfahren, mit dem Unterschied, dass anstelle von Tris-(4-methoxyphenyl) phosphin die gleiche molare Menge Triphenylphosphin eingesetzt wurde. Nach einer Reaktionsdauer von 15,5 h bei 15 bar CO-Druck wurde mittels GC die Zusammensetzung der gelösten Produkte in der Xylol-Phase bestimmt. Gefunden wurden nur 43,2% des gewünschten Produkts, aber 56,8% unumgesetztes Edukt.

**Vergleichsbeispiel 2**

Es wurde verfahren wie in Beispiel 3 beschrieben, jedoch wurde anstelle von Tris(4-methoxyphenyl)phosphin die gleiche molare Menge Tri-*n*-butylphosphin eingesetzt. Nach einer Reaktionsdauer von 15 h bei 14 bar CO-Druck wurde mittels GC die Zusammensetzung der gelösten Produkte in der Xylol-Phase bestimmt. Gefunden wurden nur Spuren (0,4%) des gewünschten Produkts, aber 96,8% unumgesetztes Edukt.

**Vergleichsbeispiel 3**

Es wurde verfahren wie in Beispiel 3 beschrieben, jedoch wurde anstelle von Tris(4-methoxyphenyl)phosphin die gleiche molare Menge Tris(3-methoxyphenyl)phosphin eingesetzt. Nach einer Reaktionsdauer von 21 h bei 19 bar CO-Druck wurde mittels GC die Zusammensetzung der gelösten Produkte in der Xylol-Phase bestimmt. Gefunden wurden nur 53,3% des gewünschten Produkts, aber 46,7% unumgesetztes Edukt.

**Vergleichsbeispiel 4**

Es wurde verfahren wie in Beispiel 3 beschrieben, jedoch wurde anstelle von Tris(4-methoxyphenyl)phosphin die gleiche molare Menge Tris(2-methoxyphenyl)phosphin eingesetzt.
Nach einer Reaktionsdauer von 21 h bei 19 bar CO-Druck wurde mittels GC die Zusammensetzung der Xylol-Phase bestimmt. Gefunden wurde nur unumgesetztes Edukt, aber keinerlei Produkte.

**Vergleichsbeispiel 5**

Es wurde verfahren wie in Beispiel 3 beschrieben, jedoch wurde anstelle von Tris(4-methoxyphenyl)phosphin die gleiche molare Menge Tris(4-chlorphenyl)phosphin eingesetzt. Nach einer Reaktionsdauer von 21 h bei 20 bar CO-Druck wurde mittels GC die Zusammensetzung der gelösten Produkte in der Xylol-Phase bestimmt. Gefunden wurden nur 26,3% des gewünschten Produkts, aber 73,7% unumgesetztes Edukt.

**Patentansprüche**

1. Verfahren zur Herstellung von Amiden der allgemeinen Formel

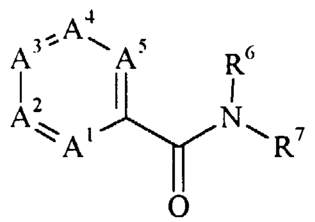

I,

worin

$A^1$ Stickstoff oder $CR^1$,
$A^2$ Stickstoff oder $CR^2$,
$A^3$ Stickstoff oder $CR^3$,
$A^4$ Stickstoff oder $CR^4$,

und $A^5$ Stickstoff oder $CR^5$ ist,
mit der Massgabe, dass wenigstens eines der Ringglieder $A^1$ bis $A^5$ Stickstoff ist und nicht zwei Stickstoffatome unmittelbar miteinander verbunden sind,

$R^1$ bis $R^5$, soweit vorhanden, unabhängig voneinander Wasserstoff, $C_{1-4}$-Alkyl oder Aryl sind, wobei einer der Substituenten $R^1$ bis $R^5$ eine Gruppe der Formel -OR ist, in welcher R ein gegebenenfalls substituierter aromatischer oder heteroaromatischer Rest ist,
$R^6$ Wasserstoff oder $C_{1-4}$-Alkyl
und $R^7$ ein gegebenenfalls substituierter aromatischer oder heteroaromatischer Rest ist, dadurch gekennzeichnet, dass eine Halogenverbindung der allgemeinen Formel

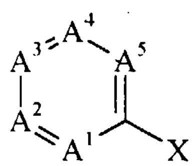

II,

worin $A^1$ bis $A^5$ die oben genannten Bedeutungen haben und X Chlor, Brom oder Iod ist, mit Kohlenmonoxid und einem Amin der allgemeinen Formel

$$R^6\text{-NH-}R^7 \qquad \text{III,}$$

worin $R^6$ und $R^7$ die oben genannten Bedeutungen haben, in Gegenwart eines Komplexes von Palladium mit einem Triphenylphosphin der allgemeinen Formel

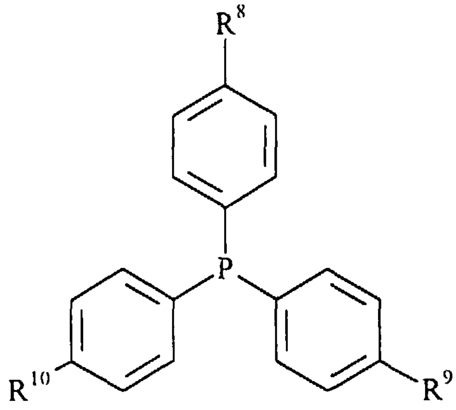

IV,

worin $R^8$ bis $R^{10}$ unabhängig voneinander $C_{1-4}$-Alkyl, $C_{1-4}$-Alkoxy oder Fluor bedeuten,

und einer Base zur Reaktion gebracht wird.

2. Verfahren nach Anspruch 1, dadurch gekennzeichnet, dass $A^2$ Stickstoff und Teil eines Pyridinringes ist.

3. Verfahren nach Anspruch 2, dadurch gekennzeichnet, dass $R^1$ eine Gruppe der Formel -OR ist, wobei R die in Anspruch 1 genannte Bedeutung hat.

4. Verfahren nach Anspruch 1, dadurch gekennzeichnet, dass $A^1$ Stickstoff und Teil eines Pyridinringes ist.

5. Verfahren nach Anspruch 1, dadurch gekennzeichnet, dass $A^1$ und $A^5$ Stickstoff und Teil eines Pyrimidinringes sind.

6. Verfahren nach Anspruch 1, dadurch gekennzeichnet, dass $A^1$ und $A^4$ Stickstoff und Teil eines Pyrazinringes sind.

7. Verfahren nach Anspruch 1, dadurch gekennzeichnet, dass $A^1$, $A^3$ und $A^5$ Stickstoff sind.

8. Verfahren nach Anspruch 4, 5, 6 oder 7, dadurch gekennzeichnet, dass $R^2$ eine Gruppe der Formel -OR ist, wobei R die in Anspruch 1 genannte Bedeutung hat.

9. Verfahren nach Anspruch 3 oder 8, dadurch gekennzeichnet, dass R eine gegebenenfalls substituierte Phenylgruppe ist.

10. Verfahren nach einem der Ansprüche 1 bis 9, dadurch gekennzeichnet, dass $R^6$ Wasserstoff und $R^7$ eine gegebenenfalls substituierte Phenylgruppe ist.

11. Verfahren nach einem der Ansprüche 1 bis 10, dadurch gekennzeichnet, dass X Chlor ist.

12. Verfahren nach einem der Ansprüche 1 bis 11, dadurch gekennzeichnet, dass $R^8$ bis $R^{10}$ gleich und $C_{1-4}$-Alkoxygruppen sind.

13. Verfahren nach Anspruch 12, dadurch gekennzeichnet, dass $R^8$ bis $R^{10}$ Methoxygruppen sind.

14. Verfahren nach Anspruch 1, dadurch gekennzeichnet, dass in einer ersten Stufe ein Dihalogenid der allgemeinen Formel

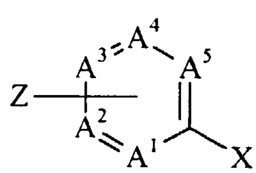

V,

worin $A^1$ bis $A^5$ die in Anspruch 1 genannten Bedeutungen haben, X Chlor, Brom oder Iod ist, einer der Reste $R^1$ bis $R^5$ an einem zu einem Ringstickstoffatom benachbarten Kohlenstoffatom Z ist, wobei Z Chlor, Brom oder Iod bedeutet und die übrigen von $R^1$ bis $R^5$, soweit vorhanden, die in Anspruch 1 genannte Bedeutung haben, mit einer aromatischen oder heteroaromatischen Hydroxyverbindung der allgemeinen Formel

R-OH VI,

worin R die in Anspruch 1 genannte Bedeutung hat, zu einer (Hetero-) Aryloxy-Halogenverbindung der allgemeinen Formel

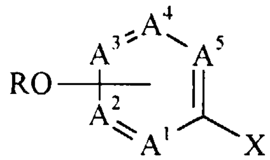

II´,

worin $A^1$ bis $A^5$, R und X die oben genannten Bedeutungen haben, umgesetzt und in einer zweiten Stufe mit Kohlenmonoxid und einem Amin der obigen allgemeinen Formel III in Gegenwart eines Komplexes von Palladium mit einem Triphenylphosphin der obigen allgemeinen Formel IV und einer Base zur Reaktion gebracht wird.

**15.** 2-Chlor-6-[1-methyl-3-(trifluormethyl)pyrazol-5-yloxy]pyridin der Formel

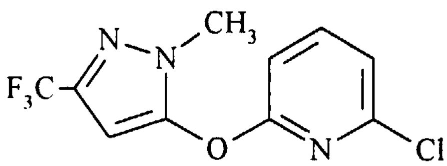

als Zwischenprodukt im Verfahren nach Anspruch 14.

## Claims

**1.** A process for the preparation of amides of the general formula:

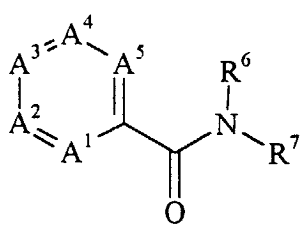

I,

wherein:

$A^1$ is nitrogen or $CR^1$,
$A^2$ is nitrogen or $CR^2$,
$A^3$ is nitrogen or $CR^3$,
$A^4$ is nitrogen or $CR^4$, and
$A^5$ is nitrogen or $CR^5$,

with the proviso that at least one of the ring members $A^1$ to $A^5$ is nitrogen and that no two nitrogen atoms are

bonded directly to one another,

$R^1$ to $R^5$, if present, independently of one another are hydrogen, $C_{1-4}$-alkyl or aryl, wherein one of the substituents $R^1$ to $R^5$ is a group of the formula -OR, in which R is an optionally substituted aromatic or heteroaromatic radical,
$R^6$ is hydrogen or $C_{1-4}$-alkyl, and
$R^7$ is an optionally substituted aromatic or heteroaromatic radical,

characterized in that a halogen compound of the general formula:

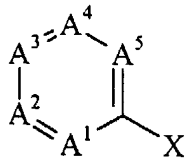

II,

in which $A^1$ to $A^5$ are as defined above and X is chlorine, bromine or iodine, is reacted with carbon monoxide and an amine of the general formula:

$$R^6\text{-NH-}R^7 \quad \text{III,}$$

in which $R^6$ and $R^7$ are as defined above,
in the presence of:
a complex of palladium with a triphenylphosphine of the general formula:

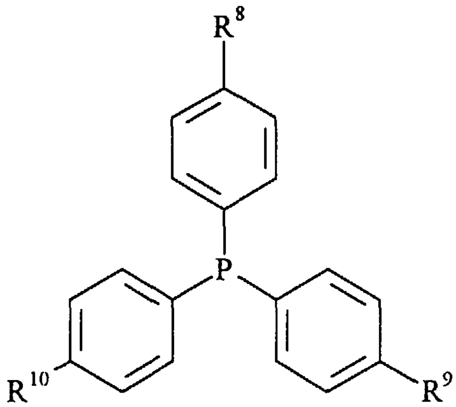

IV,

in which $R^8$ to $R^{10}$ independently of one another are $C_{1-4}$-alkyl, $C_{1-4}$-alkoxy or fluorine,
and a base.

**2.** The process according to claim 1, characterized in that $A^2$ is nitrogen and part of a pyridine ring.

**3.** The process according to claim 2, characterized in that $R^1$ is a group of the formula -OR, wherein R is as defined in claim 1.

**4.** The process according to claim 1, characterized in that $A^1$ is nitrogen and part of a pyridine ring.

**5.** The process according to claim 1, characterized in that $A^1$ and $A^5$ are nitrogen and part of a pyrimidine ring.

**6.** The process according to claim 1, characterized in that $A^1$ and $A^4$ are nitrogen and part of a pyrazine ring.

**7.** The process according to claim 1, characterized in that $A^1$, $A^3$ and $A^5$ are nitrogen.

**8.** The process according to claim 4, 5, 6 or 7, characterized in that $R^2$ is a group of the formula -OR, wherein R is as defined in claim 1.

**9.** The process according to claim 3 or 8, characterized in that R is an optionally substituted phenyl group.

**10.** The process according to one of claims 1 to 9, characterized in that $R^6$ is hydrogen and $R^7$ is an optionally substituted phenyl group.

**11.** The process according to one of claims 1 to 10, characterized in that X is chlorine.

**12.** The process according to one of claims 1 to 11, characterized in that $R^8$ to $R^{10}$ are identical and are $C_{1-4}$-alkoxy groups.

**13.** The process according to claim 12, characterized in that $R^8$ to $R^{10}$ are methoxy groups.

**14.** The process according to claim 1, characterized in that, in a first step, a dihalide of the general formula:

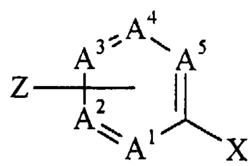

V,

wherein $A^1$ to $A^5$ are as defined in claim 1, X is chlorine, bromine or iodine, one of the radicals $R^1$ to $R^5$ on a carbon atom adjacent to a ring nitrogen atom is Z, Z being chlorine, bromine or iodine, and the remaining radicals $R^1$ to $R^5$, if present, are as defined in claim 1,
is reacted with an aromatic or heteroaromatic hydroxyl compound of the general formula:

R-OH VI,

wherein R is as defined in claim 1, to give a (hetero)aryloxy halogen compound of the general formula:

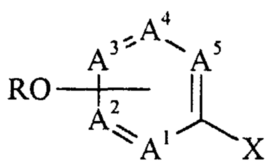

II′,

wherein $A^1$ to $A^5$, R and X are as defined above,
and, in a second step, is reacted with carbon monoxide and an amine of the above general formula III in the presence of a complex of palladium with a triphenylphosphine of the above general formula IV, and a base.

**15.** 2-Chloro-6-[1-methyl-3-(trifluoromethyl)pyrazol-5-yl-oxy]pyridine of the formula:

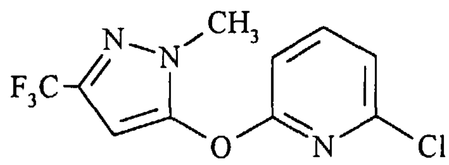

as an intermediate product in the process according to claim 14.

**Revendications**

**1.** Procédé pour la préparation d'amides de la formulé générale

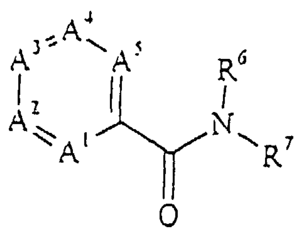

I,

dans laquelle

$A^1$ est l'azote ou $CR^1$
$A^2$ est l'azote ou $CR^2$
$A^3$ est l'azote ou $CR^3$
$A^4$ est l'azote ou $CR^4$,

et $A^5$ est l'azote ou $CR^5$,

à condition qu'au moins l'un des éléments de cycles $A^1$ jusqu'à $A^5$ soit l'azote et que deux atomes d'azote ne soient pas liés directement entre eux,
$R^1$ à $R^5$, dans la mesure où ils sont présents, sont indépendamment l'un de l'autre l'hydrogène, alkyle $C_{1-4}$- ou aryle, l'un des substituants $R^1$ à $R^5$ étant un groupe de la formule -OR, dans laquelle R est un radical hétéroaromatique ou aromatique éventuellement substitué,
$R^6$ est l'hydrogène ou alkyle $C_{1-4}$
et $R^7$ est un radical aromatique ou hétéroaromatique éventuellement substitué, caractérisé en ce que l'on met en réaction un composé halogéné de la formule générale

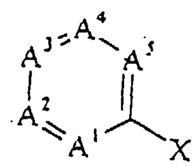

II,

dans laquelle $A^1$ à $A^5$ ont les significations ci-dessus et X représente le chlore, le brome ou l'iode, avec un monoxyde de carbone et une amine de la formule générale

$$R^6\text{-NR-}R^7 \qquad \text{III}$$

dans laquelle $R^6$ et $R^7$ ont les significations précitées, en présence d'un complexe de palladium avec une triphénylphosphine de la formule générale

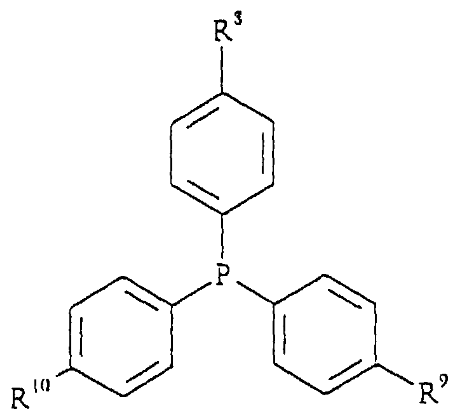

IV,

dans laquelle $R^8$ à $R^{10}$ signifient indépendamment l'un de l'autre alkyle $C_{1-4}$, alcoxy $C_{1-4}$ ou fluor,

et une base.

**2.** Procédé selon la revendication 1, caractérisé en ce que $A^2$ est l'azote et une partie d'un cycle pyridine.

**3.** Procédé selon la revendication 2, caractérisé en ce que $R^1$ est un groupe de la formule OR, R ayant la signification citée dans la revendication 1.

**4.** Procédé selon la revendication 1, caractérisé en ce que $A^1$ est l'azote et une partie d'un cycle pyridine.

**5.** Procédé selon la revendication 1, caractérisé en ce que $A^1$ et $A^5$ sont l'azote et une partie d'un cycle pyrimidine.

**6.** Procédé selon la revendication 1, caractérisé en ce que $A^1$ et $A^4$ sont l'azote et une partie d'un cycle pyrazine.

**7.** Procédé selon la revendication 1, caractérisé en ce que $A^1$, $A^3$ et $A^5$ sont l'azote.

**8.** Procédé selon la revendication 4, 5, 6 ou 7, caractérisé en ce que $R^2$ est un groupe de la formule OR, R ayant la signification citée dans la revendication 1.

**9.** Procédé selon la revendication 3 ou 8, caractérisé en ce que R est un groupe phényle éventuellement substitué.

**10.** Procédé selon l'une des revendications 1 à 9, caractérisé en ce que $R^6$ est l'hydrogène et $R^7$ un groupe phényle éventuellement substitué.

**11.** Procédé selon l'une des revendications 1 à 10, caractérisé en ce que X est le chlore.

**12.** Procédé selon l'une des revendications 1 à 11, caractérisé en ce que $R^8$ à $R^{10}$ sont identiques et sont des groupes alcoxy $C_{1-4}$.

**13.** Procédé selon la revendication 12, caractérisé en ce que $R^8$ à $R^{10}$ sont des groupes méthoxy.

**14.** Procédé selon la revendication 1, caractérisé en ce que dans une première étape on met en réaction un dihalogénure de la formule générale

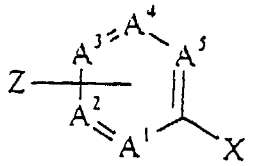

V,

dans laquelle $A^1$ à $A^5$ ont les significations citées dans la revendication 1, X est le chlore, le brome ou l'iode, l'un des radicaux $R^1$ à $R^5$ est un atome de carbone Z limitrophe d'un atome d'azote cyclique, Z signifiant le chlore, le brome ou l'iode et $R^1$ à $R^5$, dans la mesure où ils sont présent ont la signification citée dans la revendication 1, avec un composé hydroxy aromatique ou hétéroaromatique de la formule générale

R-OH VI

dans laquelle R a la signification indiquée dans la revendication 1, en un composé halogéné (hétéro-)aryloxy de la formule générale

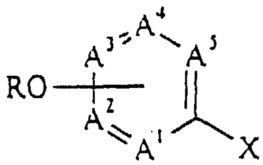

II',

dans laquelle $A^1$ à $A^5$, R et X ont les significations précitées et dans une deuxième étape on met en réaction avec un monoxyde de carbone et une amine de la formule générale ci-dessus III en présence d'un complexe de palladium avec une triphénylphosphine de la formule générale ci-dessus 4 et une base.

**15.** 2-chore-6-[1-méthyl-3-(trifluorméthyl)pyrazol-5-yloxy]pyridine de la formule

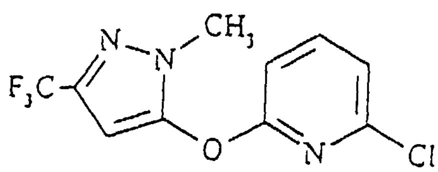

comme produit intermédiaire dans le procédé selon la revendication 14.